# EUROPEAN PATENT SPECIFICATION

(11) **EP 2 265 173 B1**
(45) Date of publication and mention of the grant of the patent: **11.06.2014**
(21) Application number: 09731757.2
(22) Date of filing: 09.04.2009
(51) Int. Cl.: A61B 5/0456, A61B 5/11

(54) **METHOD AND SYSTEM FOR SLEEP/WAKE CONDITION ESTIMATION**
VERFAHREN UND SYSTEM ZUR SCHLAF-/WACHBEDINGUNGSSCHÄTZUNG
PROCÉDÉ ET SYSTÈME D'ESTIMATION DE LA CONDITION DE VEILLE/SOMMEIL

(30) Priority: 16.04.2008 EP 08103563
(43) Date of publication of application: 29.12.2010
(73) Proprietor: Philips Intellectual Property & Standards GmbH, 20099 Hamburg (DE); Koninklijke Philips N.V., 5656 AE Eindhoven (NL)
(72) Inventor: NAUJOKAT, Elke, NL-5656 AE Eindhoven (NL); KREMER, Frederique, NL-5656 AE Eindhoven (NL); DEVOT, Sandrine, M., L., NL-5656 AE Eindhoven (NL); AUBERT, Xavier, L., M., A., NL-5656 AE Eindhoven (NL)
(74) Representative: Kroeze, Johannes Antonius
(86) International application number: PCT/IB2009/051502
(87) International publication number: WO 2009/128000

(56) References cited:
- WO-A-03/099114
- WO-A-2006/048852
- JP-A- 2000 000 214
- US-A1- 2006 235 315

## Description

### FIELD OF THE INVENTION

The invention relates to the field of multi-parameter analysis of a patient signal, and especially to the field of sleep and wake stages detection and sleep efficiency estimation, i.e. the ratio of total sleep time to total time in bed.

### BACKGROUND OF THE INVENTION

Sleep disorders are common. At least 10% of the population suffers from a sleep disorder that is clinically significant and of public health importance. Insomnia is by far the most common form of sleep disturbance.

Most insomnia definitions include descriptions of sleep-specific symptoms with associated daytime complaints. Sleep symptoms typically include a difficulty initiating sleep, difficulty maintaining sleep, final awakening that occurs much earlier than desired or sleep that is non-restorative or of generally poor quality. Waking symptoms are associated with daytime complaints related to fatigue, sleepiness, mood disturbance, cognitive difficulties and social or occupational impairment.

The prevalence of isolated insomnia symptoms in the general population is approximately 30 to 50%, and approximately 9 to 15% report significant daytime impairments as a result of chronic insomnia problems. In a majority of the patient population, insomnia is treated by medication. Other forms of treatment such as sleep restriction therapy are less wide-spread although evidence suggests that they are more effective on the long run than pharmaceutical treatment alone.

As standard diagnostic method for assessing the nature and the severity of the sleep problem, a so-called sleep log or sleep diary, i.e. a questionnaire usually on paper, is used in most cases; also an actigraph can be used as an alternative to a sleep log.

The main drawback of this sleep log is that its accuracy is affected by a subjective bias of the patient, e.g. for patients it is often difficult to remember sleep and wake periods during the night correctly.

An automatic detection of sleep and wake stages requires the measurement of vital body signs, but unfortunately most of the existing solutions rely on adhesive electrodes e.g. by sticking or gluing them to the patient's skin to wear electrodes e.g. for EEG on the head. Those electrodes are obtrusive to the patient during sleeping due to the cables and recording arrangements, which are connected to those sensors. Additionally, there is a problem that during resting time, cables or electrodes might loose the connection, which lowers the quality of the received signal.

The diagnosis of insomnia with an automatic sleep log in fully equipped sleep laboratory is expensive and capacities, places are not available over long time duration and thus ability for diagnosis for insomnia patients are limited.

Further, additional sensors and cabling along the patient may disturb the patient's sleep, and therefore influence the evaluation of sleep and wake stages during the night time, which leads to an influence on the sleep records and might lead to a wrong diagnosis.

US 2004/0225179 discloses automated behavioral methods and systems for treating insomnia that use passive means for determining wake/sleep states.

WO 2006/048852 A1 discloses a sleep monitoring system including an ECG device and a respiration inductance plenthysmogram which monitors cardiac activity and physical respiration. The representative signals are sent to a processor. It is disclosed that the respiration frequency can be derived from the ECG signal.

US 2006/0235315 disclose a method of determining sleep stages from signals of electrical activity recorded of a chest of a sleeping person. A time frequency decomposition is obtained from the series of cardiac R-R intervals, which are extracted from the electrocardiogram (ECG) signals of the patient. Slow wave sleep (SWS) and non-slow wave sleep (NSWS) periods of the patient is determined using the time frequency decomposition.

JP 2000000214 A1 discloses to detect the body movement of a person and extracting one signal of heart rate, respiration rate. To detect the heart rate an air mat is arranged under the body and the pressure inside of the mat is detected by a fine differential pressure sensor.

WO 03/099114 A1 discloses procedure for deriving reliable information on respiratory activity from heart period measurement. The respiratory frequency is determined from pattern of rhythmic changes in heart rate beat.

### SUMMARY OF THE INVENTION

It is the object of the present invention to provide a method and system based on multi-parameter analysis of signals for assessing sleep/wake s, which is reliable, easy to handle and especially unobtrusive to the patient.

This objective is achieved by using an arrangement for the identification of sleep and wake conditions of a subject, comprising, at least one bed-foil sensor, which is integrated in the sleeping environment of the subject, the sensor is provided to generate subject related signals, and calculation means, provided to receive the sensor signals, to extract at least one feature from these sensor signals, to classify the extracted feature, and to provide a probability indication whether the subject is awake or asleep.

Accordingly, such an arrangement for the identification of sleep and wake conditions of a subject is unobtrusive to the patient by using at least one sensor, which do not have to be applied directly on the body of the patient, but which are integrated in the sleeping environment like in textiles or in the patient's bed or bed slat; thus, the patient does not need any special treatment before or during sleeping, but sleeps as he is used to with his pyjama and covered with a blanket in his bed. As more such sensors are used, as more comfortable is the investigation for the patient and as better relate the measurement results to the "natural patient's sleep reality".

The invention is disclosed in independent claims 1, 10, 13 and 14.

In prior art a common method for sleep and wake condition detection utilises EEG signals, surprisingly it was found, that other signals like an ECG signal or a bed foil sensor generating an respiratory and body movement signal or a combination of the both provide a suitable estimation of the sleep and waking conditions. The estimation based on the both said signals also surprisingly showed suitable results by utilising electrodes which with non adhesive electrodes but with sensors in the sleeping environment of the subject even in a domestic sleeping environment.

The term "body movements" is used for any movement of the body, including physical activity (e.g. turning in bed, moving arms or legs), respiratory activity (thoracic movements) and ballistocardiography (small movements resulting from mechanical heart activity).

The term "sleeping environment" is defined as a subject's or patient's environment, wherein the sensors are not directly adhesively provided on the patient's body in form of self adhesive electrodes or gelled electrodes that are glued directly on the head or the skin of the patient, but wherein the sensors are provided in an unobtrusive, non adhesive direct skin contact or contactless to the patient i.e. electrodes which are provided in bed textiles or pyjamas, sleeping suits etc. Additionally, the sensors can be provided as feet mats or body mats on which the patient is lying or is being laid on, or additionally further any sensing arrangement can be used i.e. cameras, movement sensors, induction coils, etc.

Further the arrangement for the identification of sleep and wake conditions of a subject may include the sensing of an ECG signal, a signal related to body movements, a slat sensor, an inductive/piezo-resistive band, a combination of the latter. Wherein the signal extraction of the patient related sensor measuring the ECG signal includes, pulse condition, statistical heart rate variability parameters from the time domain, parameters from the heart rate variability spectrum, multi-scale sample entropy, and progressive detrended fluctuation analysis. The signal extraction of the patient related sensor signal measuring body movements includes respiratory features, e.g. mean breathing rate, activity index, cardiac features, and time and frequency domains heart rate variability parameters.

The arrangement may utilise the combination of the ECG sensor signal and the bed foil signal together or alone from unobtrusive sensors in a sleeping environment and surprisingly obtains a suitable probability estimation for the sleep and wake condition of a subject, which is normally obtain with obtrusive EEC adhesive sensors in a sleep laboratory.

The present invention therefore achieves an easy, unobtrusive and at same time reliable arrangement to obtain probability information about the sleep and wake condition of a subject even in a mobile bed, in domestic or hospital environment that is conventionally only available in a stationary bed in a sleep laboratory.

Further, the arrangement is made reliable by using a sophisticated signal processing, which allows identifying the sleep and wake condition of the patient reliably and without the need of additionally using e.g. a questionnaire.

By not involving the patient actively by e.g. filling in a questionnaire, but by unobtrusive sensing of ECG signals, and body movements signals recorded with the help of bed-foil sensors implemented arranged in T-shirts, pyjamas, blankets, and bed textiles or bed slat, the patient does not recognize the monitoring, and this allows the patient to sleep in a natural and convenient way during the night in his usual home environment. This eases monitoring of sleep and rest of healthy people as well as insomnia patients in real-time and over long terms. The present invention provides an automatic sleep log, based on multi-parameter sleep/wake estimation, which conventionally only can be achieved in fully equipped sleep laboratories

This allows further providing treatment for insomnia patient or to any patient suffering of sleeplessness of the inability to sleep for longer terms during the night without interruption.

Additionally this allows recognising sleep and wake condition of elderly people in nursing houses or at home with telemedicine facilities, in order to provide a better and economical care during night and daytime care.

According to an embodiment of the invention, the ECG signal provided by a ECG sensor undergoes a pre-processing, wherein the preprocessing includes in R-peak detection, ectopic beat removal, linear interpolation and resampling to predefined frequency, preferably at 4 Hz, and resulting in a RR-peak interval series and respiration sensing signal provided by bed foil sensor, the slat sensor any sensor which is used to sense body movements of a patient undergoes a pre-processing which consists in noise reduction. The use of low pass filtering leads to the breathing signal. Peak identification on this signal allows us to deduce the breath interval series, which is also linearly interpolated and resampling to predefined frequency, also preferably at 4 Hz.

In alternative embodiments, the RR-peak interval series is assessed on heart rate variability standards in frequency and time domains.

In further embodiments wherein a power spectrum calculation is calculated over the predefined first time duration epoch, i.e. preferably five minute, centred on a second predefined time duration epoch of interest, is computed using an autoregressive model with advanced detrending. The calculation of the power spectrum is obtained and further the power spectrum is divided into a low-frequency band which is the preferred between 0.04-0.15 Hz and into a higher frequency band which is preferred between 0.15-0.4 Hz and wherein the power spectrum is normalised. Then the extraction of non-linear parameters is provided through a progressive detrended fluctuation analysis and the calculation of the multiscale sample entropy. Additionally further, the RR-peak interval and the breath interval series are combined by estimating the squared coherence function over predefined first time duration epoch centred on a second time duration epoch i.e. preferably one minute.

The present invention also refers to a method for the identification of sleep and wake conditions of a subject comprising the following steps, to place a bed-foil sensor integrated in a sleeping environment, which is provided to generate subject related signals, to receive the sensor signals, to extract at least one feature from these sensor signals, to classify the extracted feature, and to provide a probability indication whether the subject is awake or asleep.

Further, the claim 10 relates to the obtaining of a sensor signal in the sleeping environment which allows only by obtaining a bed foil sensor signal or the combination of said signal with a ECG sensor signal or any other sensor providing a sensing of the cardio-respiratory status of the subject in using said method to achieve a probability indication of reliability whether the subject is awake or asleep.

The method of the present invention provides several step of processing, wherein the steps sensing and of the calculation including reception, extraction, and classifying an which can run automatically, and also can be scheduled repeatedly, parallel or serially.

Further it is mentioned, that present invention can be embodied in a computer programme having a programme code according to method of claim 10, when the computer programme runs on a computer and wherein the computer programme is stored on and run from a data carrier having a programme code for performing the method as claimed in claim 10, when the computer programme runs on a computer.

The "bed foil sensor" may also be combined with a slat sensor, an inductive/piezo-resistive band, or any other sensor which is adapted to sense movements.

The term "sleep/wake classification" refers to the classification of the epoch of interest as "wake" or "sleep", resulting from the probability of belonging to the respective class, or the classification "true" or "false" given as output of the classifier but also refers to the display of the classifier results on a user interface or any arrangement.

Additionally , the term "pNN50" refers to the percentage of the number of interval differences of adjacent NN-Intervals greater than 50 ms, the term "SDNN" refers to the Standard deviation of all NN-Intervals, the term "SDSD" refers to the Standard deviation between distances between adjacent intervals, the term "RR_mean" refers to mean duration of the RR-Intervals, the term "HR_mean" refers to the mean heart rate, the term "LF" refers to the low frequency range, as defined by heart rate variability standards, the term "HF" refers to the high frequency range, as defined by heart rate variability standards, the term "RMSSD"- refers to root mean square successive differences, and the term "HRV" refers to heart rate variability.

One of ordinary skill in the art will appreciate many variations and modifications within the scope of this invention. This method and system will be used for patients in hotels or at home, for mobile patient in hospital environment, as well as there are also applications possible for hospitalized patients. Also arrangements could make use of this invention that are intended for healthy persons or even animals.

It is to be noted that in this context the term "patient" or "subject" does not only apply on human beings, but also on animals. Further, the term "patient" does not mean that the respective person/animal is disease ridden, thus, also healthy people will be referred to as "patients".

### BRIEF DESCRIPTION OF THE DRAWINGS

These and other aspects of the invention will be apparent from and elucidated with reference to the embodiments described hereinafter.

In the drawings:
Fig. 1 schematically shows the general principle of the invention;
Fig. 2 shows a schematic flowchart of the feature extraction method of a first preferred embodiment of the invention;
Fig. 3 shows a schematic flowchart of the sleep/wake classification method of a first preferred embodiment of the invention;
Fig. 4 shows the sleep restriction algorithm based on the method of the present invention;
Fig. 5 schematically shows the general principle of a ferro-electret foil sensor; and
Fig. 6 shows an practical example of an ECG sensor integrated pillow and foot map.

### DETAILED DESCRIPTION OF EMBODIMENTS

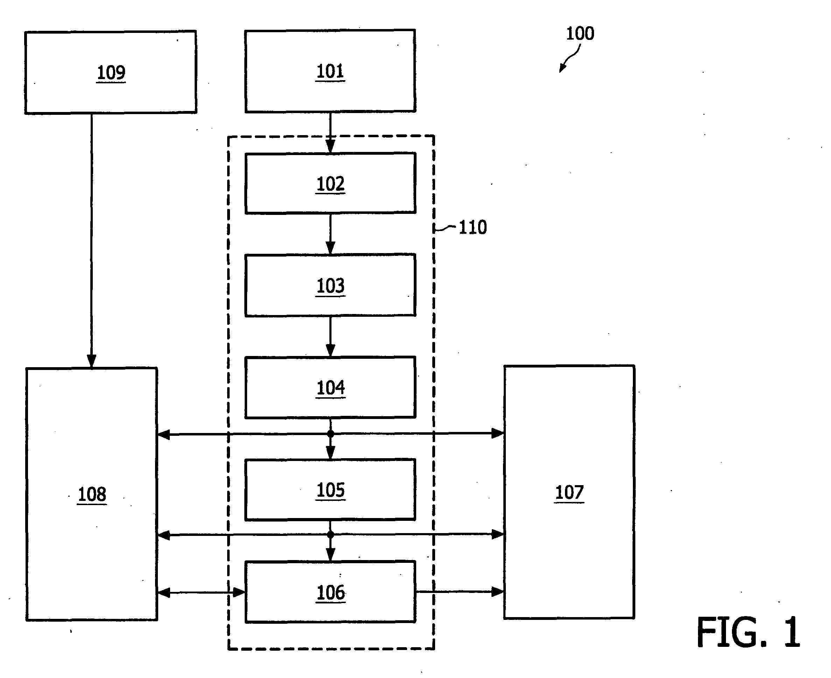

As can be seen from Figure 1, the system for sleep/wake classification **100** according to the preferred embodiment of the present invention comprises unobtrusive in-bed sensors for vital body sign monitoring **101** of the heart activity from the ECG, and the body movements from a bed foil sensor as will be described in the following. A further step is the pre-processing unit **102** for filtering and artefact removal during preparation of the signals, a feature extraction unit **103** extracting in particular features from the ECG and/or the body movements signal alone or in combination, then there is a sleep/wake classifier unit **104** for classification of the sleep /wake status according to all the input features, sleep efficiency calculation unit **105** for calculating the time asleep compared to the time in bed, used as input for the patient sleep restriction algorithm, arrangement **106** providing for example rules for a healthier sleep to the patient.

The output of the sleep/wake classification **104,** sleep efficiency calculation **105** and sleep restriction algorithm arrangement **106** can be used to provide feedback to the patient **107** or to the medical professional, who could also get information from additional sources like the sleep log questions for subjective parameters **109.**

As for the sensor part of the proposed system, the following embodiments are possible:
The bed-foil sensor may be a ferro-electret foil placed underneath the patient's thorax to measure heart rate, respiration and body movements.

In another preferred embodiment a piezo-resistive strain gauge glued onto a slat underneath the mattress in the patient's thorax region can be used to measure heart rate, respiration and body movements.

In another preferred embodiment an ECG would be used, preferably a textile ECG integrated as a pillow and foot mat electrode in the bed.

Instead of the ferro-electret foil or the strain gauge, the ECG sensor could be combined with a respiration signal, measured with a standard (inductive or piezo-resistive) band around the thorax and / or the abdomen. This type of sensor can be also integrated into textiles (e.g. a T-shirt) to make it more unobtrusive.

Alternatively, in another embodiment instead of the ferro-electret foil or the strain gauge, the ECG sensor could be combined with an accelerometer signal. The arrangement can be a wrist-worn arrangement, but preferably a 2D or 3D accelerometer is placed on the patient's trunk to measure body movements. Also this type of sensor can be integrated into textiles (e.g. a T-shirt) to make it more unobtrusive.

The processing unit comprises several steps as can be seen in figure 1. These are the preprocessing of the raw data, feature extraction and the sleep / wake classification. In the following paragraphs the different alternatives for each of these steps will be described in more detail.

The signal pre-processing arrangement **102** of figure 1 includes one or more of the following steps which can be passed through serially, parallel or repeatedly:
- appropriate filtering of the signal(s).
- artefact removal
- In case of an ECG signal, the removal of ectopic beats can be necessary.
- In case of gaps in the signal, interpolation might be necessary.

The feature extraction arrangement **103** includes extraction of features from the ECG and a respiration signal comprising:
From the ECG, the following features are derived:
   - Statistical heart rate variability parameters from the time domain (e.g. mean heart rate, SDNN, RMSSD etc.)
   - Parameters from the heart rate variability (HRV) spectrum (e.g. LF, HF)
   - Multi-scale sample entropy
   - Progressive detrended fluctuation analysis

From the respiration signal - measured by the ferro-electret foil, the slat sensor or the inductive/piezo-resistive band - the spectrum is calculated and the LF and HF power are extracted as features. In addition, the mean breathing rate is determined.

Further the feature extraction arrangement **103** allows in case of both, the ECG signal and a respiration signal are available, to calculate the coherent power of both spectra as an additional feature. Moreover, the ratio of heart rate and breathing rate can be derived as an additional feature.

Furthermore, from the ferro-electret foil or the slat sensor signal, an activity index is derived based on large body movements.

The preferred embodiment of the present invention further suggests that for the next step - the sleep/wake classification **104 -** the activity index and at least one additional feature relating to the cardiac and/or respiratory status are a preferable combination as input for the classification process.

A vector, of at least one feature, and preferably all or at least a sub-set of the above-mentioned features is generated for each epoch of interest, e.g. each 1-minute segment of data.

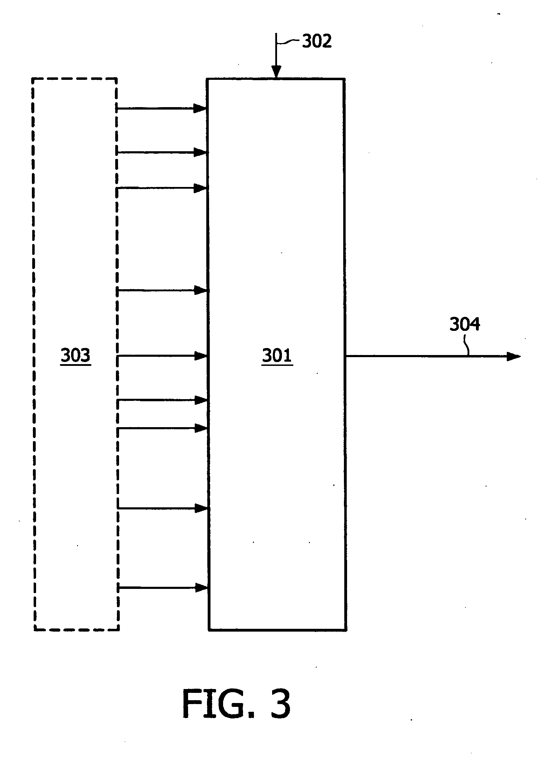

Further this vector is fed into a sleep/wake classifier **104** which is based on a standard pattern recognition approach with supervised learning, as will be described in Figure 3. For the classifier, the following approaches can preferably be used:
- Bayesian linear or quadratic discriminant classifier
- support vector machine
- k-Nearest-Neighbour (kNN) method
- Neural Network
- Hidden Markov Model

The parameters of the classifier are trained on a large database of representative data.

In order to receive the patient's input regarding the subjective questions in the sleep log and to give feedback to him, the input unit and the display unit are preferably combined in the preferred embodiment of the present invention in one user interface arrangement **107.** This arrangement can be a normal laptop PC, a tablet PC with a touch screen, a handheld arrangement such as a PDA or a mobile phone. Depending on the processing power of this arrangement, the processing unit can also be part of this arrangement. The feedback to the patient can contain one or more of the following parameters: time in bed, total sleep time, total wake time, sleep efficiency, sleep latency, number and durations of awakenings, or a simplified hypnogram.

The feedback to the medical professional can be given via the same user interface **108.** In another embodiment, there is a download procedure to transfer the patient's data from the patient user interface to the physician's PC (e.g. via a USB cable, via Bluetooth, ZigBee or any other communication standard or arrangement). In yet another embodiment, the patient's data can be (automatically) sent to the physician via the internet or GSM, UMTS, EDGE, GPRS, or any other internet or mobile phone standard or system.

The feedback to the medical professional should contain all of the above-mentioned parameters. Furthermore, it should also contain the patient's answers to the subjective sleep log questions so that he can compare the subjective and objective data which gives him important information for the appropriate therapy approach. This is for example especially important in case of sleep-state misperception, a type of insomnia where objective sleep data reflect a normal sleep pattern but the patient himself does not recognise that he has slept.

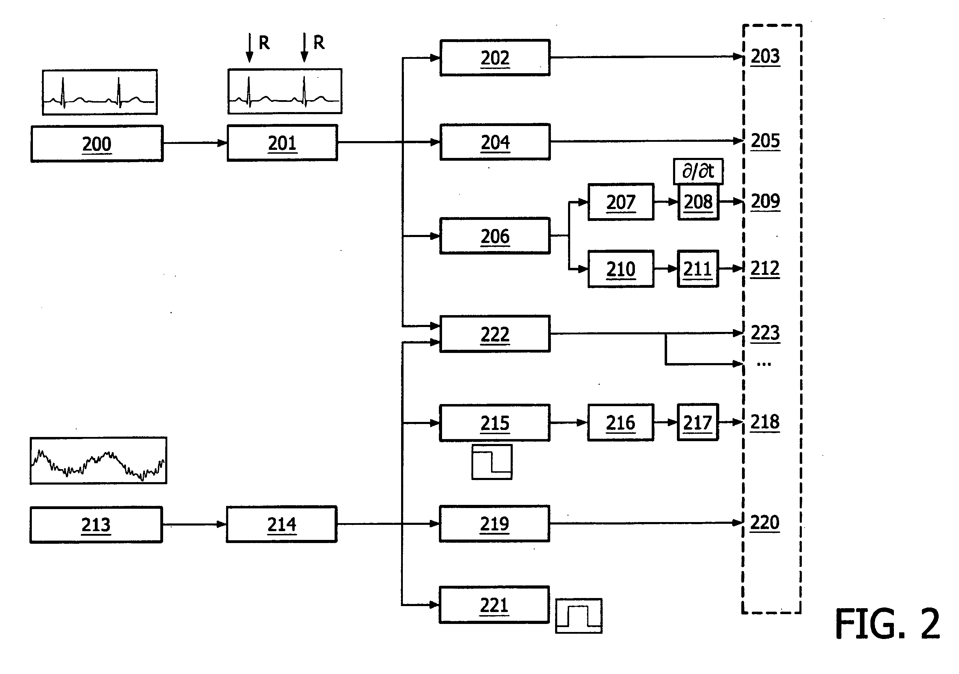

As can be seen in Figure 2, the ECG signal provided by an ECG sensor **200** undergoes a pre-processing **201,** wherein the preprocessing consists in R-peak detection, ectopic beat removal, linear interpolation and resampling at a predefined frequency, preferably at 4 Hz. The resulting RR- interval series is then considered and heart rate variability parameters, according to the standard, are assessed in frequency and time domains. First the power spectrum calculation **202** over a predefined time period, preferably a 5-minute segment of the time series, centred on the one-minute epoch of interest, is computed using preferably an autoregressive model with advanced detrending. Alternatively, other methods based on Fourier analysis, time-frequency distributions, time-varying autoregressive modeling is available. In the two last cases, the power spectral estimation is updated on a shorter time scale, e.g. every new detected R-peak of the ECG.

The power spectrum in the low-frequency band LF preferably at 0.04-0.15 Hz and in the high frequency band HF preferably at 0.15-0.4 Hz are used to define the spectral features in **203:** the power is normalised according to LF_norm=LF/(LF+HF) and LF/HF ratio is calculated. Furthermore the RR-interval series time statistics over a predefined time period, preferably a 5-minute segment, in **204** provides the resulting time domain features in **205,** e.g. pNN50 (percentage of the number of interval differences of adjacent NN-Intervals greater than 50 ms ), SDNN (Standard deviation of all NN-Intervals), SDSD (Standard deviation of successive differences between adjacent intervals), RMSSD (root mean square successive differences), RR_mean (mean duration of the RR-Intervals) and HR_mean (mean of the instantaneous heart rate).

Non-linear parameters are also extracted from the RR-interval time series in **206** by the application of two methods. The first non-linear calculation method used in **207** is progressive detrended fluctuation analysis which allows to gradually integrate the signal before detrending over windows of length 64. Further the partial sums of the squared signal are then considered and this provides the resulting differentiated time series in **208** from which we extract a new feature **209,** defined as the maximum value over the considered epoch, preferably a one-minute epoch but which can be any predefined epoch duration.

The second non-linear calculation method applied to the RR- interval series provides the multiscale sample entropy. Firstly, the series is coarse-grained at scales 1 and 2 in **210** and 5-minute segments are considered. The sample entropy (hereafter called sampen) is calculated in **211** at several levels from 1 to 10 Hence, the following features in **212** are
provided: sampen_scalel_k, for levels k=1 to 10 and sampen_scale2_k, for levels k=1 to 10.

As can be seen in the lower part of Figure 2, the bed foil signal provided by bed foil sensor **213** is undergoing a pre-processing **214** which consists in noise reduction and calibration. The use of low pass filtering **215** leads to the breathing signal. Peak identification on this signal allows to deduce the breath interval series, which is also linearly interpolated and resampled at a predefined rate, preferably at 4 Hz in **216.** The power spectrum **217** is computed using preferably an autoregressive model with advanced detrending. The power spectrum is then split and normalised in **218** in the low-frequency band LF (0.04-0.15 Hz) and in the high frequency band HF (0.15-0.4 Hz), which are used to define the spectral features LF_norm_respi and LF/HF ratio_respi. Besides, the detection of small and large energy artefacts in **219** allows to define a heuristic activity index over one-minute epochs, also used as a feature in **220.** Further, the band-pass filtering in **221** delivers the so-called ballistocardiogram, which represents the mechanical heart activity. This signal could be an interesting alternative to the ECG signal to get the heart rate variability signal. Finally, the RR- interval and the breath interval series are combined in **222** by estimating the squared coherence function over 5-minute epochs centred on the one-minute epoch of interest. This coherence function is multiplied by the autospectrum of the RR-interval series and integrated along the frequency axis. The resulting feature in **223** is the amount of coherent power in %. We could also think of other features assessing the cardiopulmonary coupling, like the RR-interval/ breath interval ratio.

As can be seen in the right part of Figure 2, a preferred embodiment contains the features **203, 205, 209, 212, 223, 218, 220,** which form the components of the feature vector used in the classification process which is further described in Figure 3.

Figure 3 is a preferred embodiment of the method for sleep/wake classification. Step **303** represents information coming from the feature extraction process providing a vector with at least one element, which belongs to the test data set. The decision is based on a supervised learning classifier **301,** which is trained with a training data set **302.** The classifier **304** decides on the basis of a Bayesian linear or quadratic discriminant classifier, a support vector machine or the k-Nearest-Neighbour (kNN) classifier and with a supervised learning approach based on the training data set, whether the patient is awake or asleep. The more representative the training data are, the better the accuracy and performance of classification **304.**

The sleep restriction therapy is a non-pharmacological method that can be used to treat insomnia either alone or in combination with pharmacological treatment. There is a natural tendency among poor sleepers to increase the amount of time spent in bed in an effort to provide more opportunity for sleep, a strategy that is more likely to result in fragmented and poor quality sleep.

The sleep restriction therapy consists of curtailing the amount of time spent in bed to the actual amount of time asleep. Time in bed is subsequently adjusted on the basis of the sleep efficiency calculation for a given period of time, which is usually the preceding week. For example, if a person reports sleeping an average of 6 hours per night out of 8 hours spent in bed, the initial prescribed sleep window would be 6 hours.

The subsequent allowable time in bed by about 15 to 20 minutes for a given week when sleep efficiency exceeds 85%, decreased by the same amount of time when the sleep efficiency is lower than 80% and kept stable when the sleep efficiency falls between 80 and 85%. Adjustments are made periodically, for example weekly, until optimal sleep duration is achieved. Variations in implementing this procedure may involve changing the time in bed on the basis of a moving average of the sleep efficiency, for example, of the past three to five days, or changing it on a weekly basis regardless of change of the sleep efficiency. This procedure improves sleep continuity through a mild sleep deprivation and a reduction of sleep anticipatory anxiety. To prevent excessive daytime sleepiness, the time in bed should not be reduced to less than five hours per night.

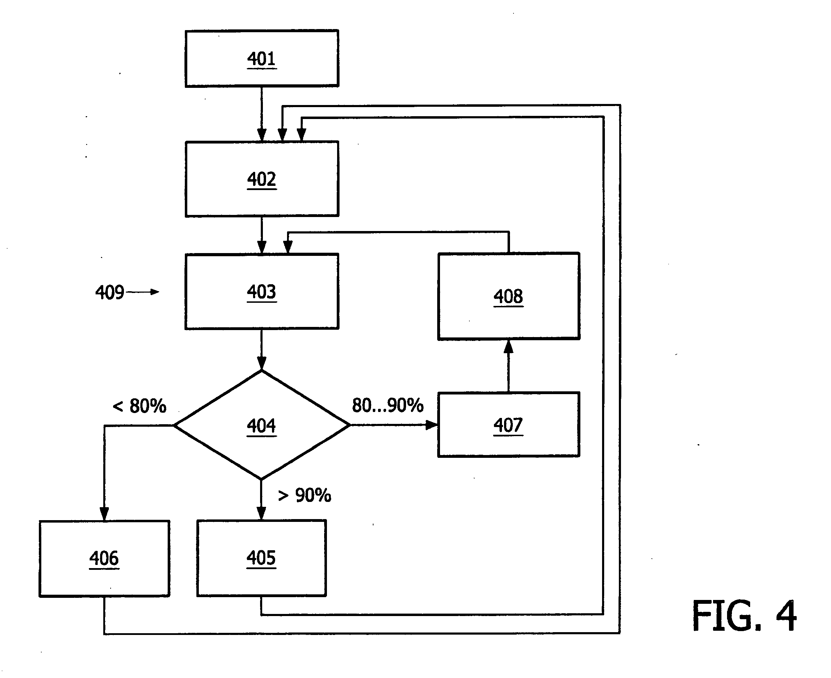

As can be seen in Figure 4, there is a preferred embodiment of the sleep restriction therapy method based on the sleep efficiency calculation which is provided by the present invention.

In the first step **401,** the medical practitioner initialises wake-up-time and go-to-bed time. In step **402** data is collected for five days, which also includes information from questionnaires.

In step **403** the mean sleep efficiency of the last five days is calculated. In step **404** a case decision is made, deciding whether the mean sleep efficiency is lower than 80%, 80 to 90% or greater than 90%.

In case the mean sleep efficiency is lower than 80%, in step **406,** the patient is asked to shorten the time in bed by 15 minutes, in case the mean sleep efficiency is greater than 90 %, in step **405** the patient is asked to prolong time in bed by 15 minutes. The process recontinues at step **402** with the collection of sleep information for the next five days. In case the mean sleep efficiency is between 80 to 90%, in step **407** positive feedback is given to the patient and in step **408** the wake-up-time and the go-to-bed time will be kept for one more day and the process continues at step **403.**

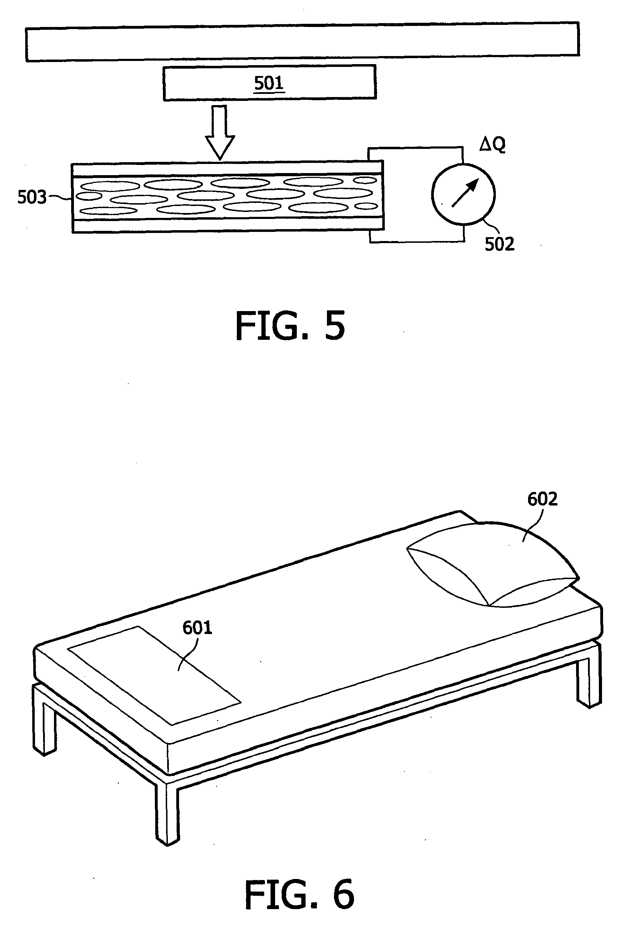

As can be seen in Figure 5, there is a schematic diagram showing the principle of an ferro-electret foil **503** as a suitable embodiment of the bed foil sensor, wherein the change of force **501**due to body movement or respiration is sensed by to the ferro-electret foil **502,** in a shifting of charges, relating to said movement forces

Figure 6 shows a practical embodiment of a conventional bed for sensing the ECG during the patient sleeping in a usual way. A textile ECG sensor consisting of a foot mat electrode **601** and a pillow **602** made of conductive material acting as electrodes which record the electrical activity of the heart.

One of ordinary skill in the art will appreciate many variations and modifications within the scope of this invention. This method and system will be used mainly for preferably insomnia patients at home or for patients at home or in hotels, for mobile patients in the hospital environment, during transport or at home but there are also applications possible for hospitalized patients. Also arrangements could make use of this invention that are intended for healthy persons or even animals. Further, while the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. Arrangement for the identification of sleep and wake conditions of a subject, comprising
- at least one bed-foil sensor integrated in the sleeping environment of the subject, the bed foil sensor generating an respiratory and body movement signal, wherein body movement signal comprises physical activity, respiratory activity and ballistocardiography, , and
- calculation means, provided
○ to receive the sensor signals,
○ to extract at least one feature from these sensor signals,
○ to classify the extracted feature, and
○ to provide a probability indication whether the subject is awake or asleep.

2. Arrangement according to claim 1, wherein further an ECG sensor is provided.

3. Arrangement according to claim 2, wherein the extracting of at least one feature from at least one measured signal derived from the sensor measuring the ECG signal and the bed foil sensor, which senses the body movements includes the cardio-respiratory condition and the coherent power spectrum calculated from both the ECG signal and bed foil sensor respiratory signal.

4. Arrangement according to claim 2 or 3, wherein the ECG signal provided by the ECG sensor undergoes a pre-processing, wherein the preprocessing includes a R-peak detection, ectopic beat removal, linear interpolation and resampling to predefined frequency, and resulting in a RR- interval series.

5. Arrangement according to claim 4, wherein the RR- interval series is further processed using heart rate variability standards in the frequency and in the time domain.

6. Arrangement according to claim 1 or 2, wherein a power spectrum is calculated over the predefined first time duration, centred on a second predefined time duration epoch of interest, using a detrending model.

7. Arrangement according to any of claims 3-6, wherein the power spectrum is divided into a low-frequency band and in the high frequency band and wherein the power spectrum is normalised.

8. Arrangement according to claim 4 or 5, wherein the RR- interval and the breath interval series are combined by estimating the squared coherence function over predefined first time duration centred on a second predefined time duration epoch.

9. Arrangement according to any of the above-mentioned claims, wherein the at least one feature form the components of a feature vector for classifying and the feature classification is based on a standard pattern recognition approach with supervised learning, and wherein the classifier includes
- Bayesian linear or quadratic discriminant classifier,
- support vector machine,
- k-Nearest-Neighbour (kNN) method,
- Neural Network,
- Hidden Markov Model (HMM), and
wherein the parameters of the classifier are trained on a large database of representative data.

10. Method for the identification of sleep and wake conditions of a subject comprising the following steps:
- to place a sensor integrated in a sleeping environment, which is provided to generate subject related signals,
- to receive the sensor signals,
- to extract at least one feature from these sensor signals,
- to classify the extracted feature, and
- to provide a probability indication whether the subject is awake or asleep
, wherein the at least one sensor signal in the sleeping environment is obtained from a bed foil sensor providing a sensing of the cardio-respiratory status of the subject and from the respiration signal the power spectrum is calculated and LF and HF are extracted.

11. Method according to claim 10, wherein the classification is based on a data training vector set and the data may be fed back into the classifier, and additionally training data may be provided from or compared to questionnaire data.

12. Method according to claim 11, wherein the steps sensing and of the calculation including reception, extraction, and classifying can run automatically, and can be scheduled repeatedly, parallel or serially

13. Computer programme having a programme code according to method of claim 10, when the computer programme runs on a computer.

14. Data carrier having a programme code for performing the method as claimed in claim 10, when the computer programme runs on a computer.

## Patentansprüche

1. Anordnung zur Erkennung des Schlaf- und Wachzustands eines Objekts, die Folgendes umfasst:
- zumindest ein Bett-Foliensensor, der in die Schlafumgebung des Objekts integriert ist, wobei der Bett-Foliensensor ein Signal bezüglich Atmung und Körperbewegung, wobei das Körperbewegungssignal körperliche Aktivität, Atmungsaktivität und Ballistokardiographie umfasst, und
- Rechenmittel, die dafür ausgelegt sind,
die Sensorsignale zu empfangen,
aus diesen Sensorsignalen zumindest ein Merkmal zu extrahieren,
das extrahierte Merkmal zu klassifizieren und
eine Wahrscheinlichkeitsangabe zu liefern, ob das Objekt wach ist oder schläft.

2. Anordnung nach Anspruch 1, wobei ferner ein EKG-Sensor geschaffen wird.

3. Anordnung nach Anspruch 2, wobei die Extraktion zumindest eines Merkmals aus zumindest einem gemessenen Signal, das von dem Sensor, der das EKG-Signal misst, und dem Bett-Foliensensor, der die Körperbewegungen erfasst, abgeleitet wird, den Herz- und Atemwegszustand und das kohärente Leistungsspektrum umfasst, das aus sowohl dem EKG-Signal als auch dem Atmungssignal des Bett-Foliensensors berechnet wird.

4. Anordnung nach Anspruch 2 oder 3, wobei das von dem EKG-Sensor gelieferte EKG-Signal einer Vorverarbeitung unterzogen wird, wobei die Vorverarbeitung die Erkennung einer R-Zacke, das Entfernen ektopischer Herzschläge, die lineare Interpolation und die Abtastratenkonvertierung auf eine vorbestimmte Frequenz umfasst und sich eine Folge von RR-Intervallen ergibt.

5. Anordnung nach Anspruch 4, wobei die Folge von RR-Intervallen unter Verwendung von Standards zur Herzfrequenzvariabilität im Frequenz- und im Zeitbereich weiterverarbeitet wird.

6. Anordnung nach Anspruch 1 oder 2, wobei ein Leistungsspektrum während der vorher festgelegten ersten Zeitdauer zentriert auf eine zweite vorher festgelegte Zeitdauer von Interesse unter Verwendung eines Modells der Trendbeseitigung berechnet wird.

7. Anordnung nach einem der Ansprüche 3 bis 6, wobei das Leistungsspektrum in ein Niederfrequenzband und in das Hochfrequenzband unterteilt wird und wobei das Leistungsspektrum normalisiert wird.

8. Anordnung nach Anspruch 4 oder 5, wobei die Folge von RR-Intervallen und die Folge von Atemintervallen kombiniert werden, indem die quadrierte Kohärenzfunktion während der vorher festgelegten ersten Zeitdauer zentriert auf eine zweite vorher festgelegte Zeitdauer ermittelt wird.

9. Anordnung nach einem der vorhergehenden Ansprüche, wobei das zumindest eine Merkmal die Komponenten eines Merkmalsvektors zur Klassifizierung bildet und die Merkmalsklassifizierung auf einem standardmäßigen Mustererkennungsverfahren mit überwachtem Lernen basiert und wobei zu den Klassifikatoren Folgende gehören:
- linearer oder quadratischer Diskriminanzklassifikator nach Bayes,
- Support-Vector-Machine,
- Methode des k-nächsten Nachbarn (KNN),
- neuronales Netz,
- Hidden-Markov-Model (HMM) und
wobei die Parameter des Klassifikators anhand einer großen Datenbank mit repräsentativen Daten trainiert werden.

10. Verfahren zur Erkennung von Schlaf- und Wachzuständen eines Objekts, das die folgenden Schritte umfasst:
- Platzieren eines in eine Schlafumgebung integrierten Sensors, der dafür geschaffen wird, objektbezogene Signale zu erzeugen,
- Empfangen der Sensorsignale,
- Extrahieren zumindest eines Merkmals aus diesen Sensorsignalen,
- Klassifizieren des extrahierten Merkmals und
- Liefern einer Wahrscheinlichkeitsangabe, ob das Objekt wach ist oder schläft,
wobei das zumindest eine Sensorsignal in der Schlafumgebung von einem Bett-Foliensensor erhalten wird, der eine Erfassung des Herz- und Atemwegszustands des Objekts durchführt, und wobei ausgehend von dem Atmungssignal das Leistungsspektrum berechnet wird und NF und HF extrahiert werden.

11. Verfahren nach Anspruch 10, wobei die Klassifizierung auf einem Satz mit Trainingsdatenvektoren basiert und die Daten zurück in den Klassifikator geleitet werden können sowie zusätzliche Trainingsdaten von Daten aus Fragebögen stammen oder mit diesen verglichen werden können.

12. Verfahren nach Anspruch 11, wobei die Schritte des Erfassens und des Empfangens einschließlich der Berechnung, der Extraktion und der Klassifizierung automatisch ablaufen und als wiederholt, parallel oder fortlaufend ablaufend geplant werden können.

13. Computerprogramm mit einem Programmcode gemäß dem Verfahren nach Anspruch 10, wobei das Computerprogramm auf einem Computer läuft.

14. Datenträger mit einem Programmcode zur Durchführung des Verfahrens nach Anspruch 10, wobei das Computerprogramm auf einem Computer läuft.

## Revendications

1. Agencement pour l'identification de conditions de sommeil et de réveil d'un sujet, comprenant :
- au moins un capteur en feuille de lit intégré dans l'environnement de sommeil du sujet, le capteur en feuille de lit générant un signal respiratoire et de mouvement corporel, dans lequel le signal de mouvement corporel comprend une activité physique, une activité respiratoire et une balistocardiographie, et
- des moyens de calcul, prévus
pour recevoir les signaux de capteur,
pour extraire au moins une caractéristique à partir de ces signaux de capteur,
pour classifier la caractéristique extraite, et
pour fournir une indication de probabilité que le sujet est réveillé ou endormi.

2. Agencement selon la revendication 1, dans lequel en outre un capteur ECG est prévu.

3. Agencement selon la revendication 2, dans lequel l'extraction d'au moins une caractéristique à partir d'au moins un signal mesuré dérivé du capteur mesurant le signal ECG et du capteur en feuille de lit, qui détecte les mouvements corporels, comprend la condition cardio-respiratoire et le spectre de puissance cohérente calculé à partir du signal ECG ainsi que du signal respiratoire de capteur en feuille de lit.

4. Agencement selon la revendication 2 ou 3, dans lequel le signal ECG fourni par le capteur ECG subit un prétraitement, dans lequel le prétraitement comprend une détection de pic R, une élimination de contraction ectopique, une interpolation linéaire et un ré-échantillonnage selon une fréquence prédéfinie, et entraînant une série d'intervalles RR.

5. Agencement selon la revendication 4, dans lequel la série d'intervalles RR est en outre traitée en utilisant des standards de variabilité de fréquence cardiaque dans la fréquence et dans le domaine temporel.

6. Agencement selon la revendication 1 ou 2, dans lequel un spectre de puissance est calculé sur la première durée prédéfinie, centrée sur une époque d'intérêt de seconde durée prédéfinie, en utilisant un modèle de redressement.

7. Agencement selon une quelconque des revendications 3 à 6, dans lequel le spectre de puissance est divisé en une bande à basse fréquence et en la bande à haute fréquence et dans lequel le spectre de puissance est normalisé.

8. Agencement selon la revendication 4 ou 5, dans lequel l'intervalle RR et la série d'intervalles de respiration sont combinés en estimant la fonction de cohérence au carré sur une première durée prédéfinie centrée sur une époque de seconde durée prédéfinie.

9. Agencement selon une quelconque des revendications mentionnées ci-dessus, dans lequel l'au moins une caractéristique forme les composants d'un vecteur de caractéristique pour la classification et la classification de caractéristique est fondée sur une approche de reconnaissance de profil standard avec apprentissage supervisé, et dans lequel le classificateur comprend :
- un classificateur discriminant linéaire ou quadratique bayésien,
- une machine à vecteur de support,
- un procédé du plus proche voisin k (« k-Nearest-Neighbour » ou kNN),
- un réseau neuronal,
- un modèle de Markov caché (« Hidden Markov Model » ou HMM), et
dans lequel l'apprentissage des paramètres du classificateur est réalisé sur une grande base de données de données représentatives.

10. Procédé pour l'identification de conditions de sommeil et de réveil d'un sujet comprenant les étapes suivantes :
- le placement d'un capteur intégré dans un environnement de sommeil, qui est prévu pour générer des signaux connexes à un sujet,
- la réception des signaux de capteur,
- l'extraction d'au moins une caractéristique à partir de ces signaux de capteur,
- la classification de la caractéristique extraite, et
- la fourniture d'une indication de probabilité que le sujet est réveillé ou endormi,
dans lequel l'au moins un signal de capteur dans l'environnement de sommeil est obtenu à partir d'un capteur en feuille de lit fournissant une détection de l'état cardio-respiratoire du sujet et à partir du signal de respiration le spectre de puissance est calculé et LF et HF sont extraits.

11. Procédé selon la revendication 10, dans lequel la classification est fondée sur un jeu de vecteurs d'apprentissage de données et les données peuvent être réintroduites dans le classificateur, et en outre des données d'apprentissage peuvent être fournies à partir de, ou comparées à des, données de questionnaire.

12. Procédé selon la revendication 11, dans lequel les étapes de détection et du calcul comprenant la réception, l'extraction, et la classification peuvent être exécutées automatiquement, et peuvent être programmées à plusieurs reprises, en parallèle ou en série.

13. Programme d'ordinateur comportant un code de programme selon procédé de la revendication 10, lorsque le programme d'ordinateur est exécuté sur un ordinateur.

14. Support de données comportant un code de programme pour réaliser le procédé selon la revendication 10, lorsque le programme d'ordinateur est exécuté sur un ordinateur.
